# EUROPEAN PATENT SPECIFICATION

(11) **EP 1 019 363 B1**
(45) Date of publication and mention of the grant of the patent: **28.08.2002**
(21) Application number: 97940490.2
(22) Date of filing: 23.09.1997
(51) Int. Cl.: C07C 231/16

(54) **PROCESS FOR THE PREPARATION OF AN INORGANIC SALT OF AN OPTICALLY ACTIVE PHENYLGLYCINE DERIVATIVE**
VERFAHREN ZUR HERSTELLUNG VON INORGANISCHEN SALZEN VON OPTISCH AKTIVEN PHENYLGLYCIN-DERIVATEN
PROCEDE DE PREPARATION D'UN SEL INORGANIQUE D'UN DERIVE PHENYLGLYCINE OPTIQUEMENT ACTIF

(30) Priority: 24.09.1996 BE 9600805
(43) Date of publication of application: 19.07.2000
(73) Proprietor: DSM N.V., 6411 TE Heerlen (NL)
(72) Inventor: BOONEN, Jozef, Johannes, Catharina, Jacobus, NL-5971 MA Grubbenvorst (NL)
(86) International application number: NL9700531
(87) International publication number: WO98013335

(56) References cited:
- EP-A- 0 442 584
- EP-A- 0 442 585
- US-A- 4 093 653
- US-A- 4 094 904

## Description

The invention relates to a process for the preparation of a hydrochloric acid salt of an optically active phenylglycine derivative in which in an asymmetric transformation a diastereoisomeric salt of the optically active phenylglycine derivative and an optically active acid is treated with hydrochloric acid.

Such a process is described in EP-A-442584, in which the L-mandelic acid salt of D-phenylglycineamide (D-PGA), after an asymmetric transformation reaction, is filtered off from the reaction mixture, washed and subsequently dissolved in water and converted into the D-PGA HCl salt with the aid of HCl.

The known process for converting the mandelic acid salt of D-PGA into the D-PGA.HCl salt is very complex and involves substantial losses of D-PGA and L-mandelic acid.

The aim of the invention is to provide a process that does not present the above drawbacks.

This is achieved according to the invention by adding an equivalent amount of hydrochloric acid relative to the amount of diasteroisomeric salt to a reaction mixture containing an amount of the diastereoisomeric salt as a solid substance and by reusing the mother liquor containing the optically active acid and remaining after recovery of the hydrochloric acid salt of the phenylglycine derivative, in the asymmetric transformation process.

Surprisingly, it has been found that it is possible to realize full conversion of the diastereoisomeric salt of the phenylglycine derivative and the optically active acid into the inorganic salt of the optically active phenylglycine derivative without isolation and/or dissolution of the diastereoisomeric salt and with the optical activity, to be expressed in enantiomeric excess (e.e.) being retained.

Asymmetric transformations in which one enantiomer is isolated from a racemic mixture and the other enantiomer is racemized in situ are known in the literature and are described in for example the aforementioned patent application EP-A-442584 and in US 4 093 653. For a commercially attractive process an efficient recovery of both the optically active phenylglycine derivative and the optically active acid is however of great importance. The known methods for the recovery of the optically active enantiomer and the optically active acid are complex, however. For example, in EP-A-442584 the optically active phenylglycine derivative is obtained from the isolated diastereoisomeric salt by dissolving the salt in a mixture of water and an almost equimolar amount of mineral acid such as hydrochloric acid, sulphuric acid, nitric acid or phosphoric acid and extracting the optically active carboxylic acid with the aid of an extractive-distillation agent. This process is rather laborious; it moreover often involves the loss of a significant portion of the optically active acid.

The process according to the invention is with particular advantage applied to the reaction mixture obtained after the asymmetric transformation. The invention hence relates to a process for the preparation of an optically active phenylglycine derivative in which the reaction mixture obtained after the asymmetric transformation is treated with an equivalent amount of hydrochloric acid relative to the amount of diastereoisomeric salt without the formed, precipitated diastereoisomeric salt of the optically active phenylglycine derivative and the optically active acid being isolated and dissolved beforehand and wherein the mother liquor containing the optically active acid obtained in the conversion can be returned as such to the asymmetric transformation.

Preferably, the amide or an ester of a phenylglycine is used as the phenylglycine derivative, which phenylglycine may or may not be substituted, in particular phenylglycine or p-hydroxyphenylglycine.

Use of an equivalent amount of hydrochloric acid relative to the amount of diastereoisomeric salt results in an almost quantitative conversion. In the application of the process according to the invention to the reaction mixture obtained after an asymmetric transformation, this presents the advantage that no inorganic salt is present in the mother liquor that remains after the recovery of the phenylglycine derivative. HCl salt. As a result, the mother liquor containing the optically active acid obtained in the conversion can be returned as such to the asymmetric transformation, for it has been found that the presence of inorganic acid interferes with the asymmetric transformation reaction, in particular the racemization.

The temperature at which the treatment with the hydrochloric acid takes place is not critical and preferably lies between 0 and 125°C, in particular between 20 and 80°C. In practice, when the treatment is applied to a reaction mixture obtained after an asymmetric transformation, it will usually take place at a temperature that is the same as or lower than the temperature at which the asymmetric transformation is carried out.

The pressure at which the treatment with hydrochloric acid takes place is not critical either and will usually lie between 0.01 and 1 MPa, in particular between 0.05 and 0.2 MPa. Preferably the treatment with hydrochloric acid is carried out at atmospheric pressure.

Carbonyl compounds that can be used in the asymmetric transformation are for example aldehydes or ketones, in particular aromatic aldehydes such as benzaldehyde, anisealdehyde, o-, p- or m-nitrobenzaldehyde, o-, p- or m-chlorobenzaldehyde or aliphatic aldehydes such as isobutyraldehyde or isovaleraldehyde, ketones such as methylisobutylketone, butanone or acetone. The amount of carbonyl compound to be added is preferably 0.5-4.0 equivalents relative to the amount of phenylglycine derivative, in particular 1-2 equivalents.

Instead of starting from a mixture of L- and D-phenylglycine derivatives and a carbonyl compound, it is also possible to start from a mixture of the Schiff bases of L- and D-phenylglycine derivatives. In this case it is not strictly necessary to add an extra amount of carbonyl compound. In this case, in order to obtain an optimum yield of the diastereoisomeric salt of optically active phenylglycine derivative and optically active acid, an amount of water that is at least equimolar relative to the amount of Schiff base must be added. The use of less than an equimolar amount of water leads to a virtually proportional decrease in the yield.

In the asymmetric transformation use is made of optically active acids such as carboxylic acids. Suitable examples of optically active acids are mandelic acid, tartaric acid, 2-pyrrolidone-5-carboxylic acid and N-acetylphenylglycine. The acidity (pKa) will usually be between 3 and 5. The amount of optically active acid to be used may vary within a wide range and will generally lie between 0.9 and 1.2 equivalents of optically active acid relative to the amino acid amide. Preferably, an equivalent amount of carboxylic acid is used.

Suitable solvents for the asymmetric transformation are for example hydrocarbons such as cyclohexane, heptane and octane, aromatic hydrocarbons such as toluene, xylene and benzene, ethers such as methyl-tertiary-butyl ether, dioxane, tetrahydrofuran and anisole, esters such as butyl acetate and ethyl acetate, ketones such as acetone, butanone, methylisobutylketone, carboxylic acids, aldehydes or mixtures of these substances. It will be clear that the solvent must be chosen so that it does not enter into irreversible chemical reactions with the amino acid amide, the optically active carboxylic acid or the aldehyde.

The pressure at which the asymmetric transformation is carried out is not critical and usually lies between 0.01 and 1 MPa, in particular 0.05 and 0.2 MPa. Preferably, the process is carried out at atmospheric pressure. The temperature at which the asymmetric transformation is carried out may vary within a wide range and is generally 70-120°C, preferably 75-100°C, in particular 80-90°C. The reaction time is usually 1-8 hours, preferably 1-4 hours.

The slurry concentration of the diastereoisomeric salts at the end of the reaction is about 5-30 wt.%, preferably 10-20 wt.%.

The invention will now be elucidated with reference to the following examples.

### Example I

400 grams (4 mol) of methylisobutylketone, 37.5 grams (0.25 mol) of DL-phenylglycineamide and 39.9 grams (0.263 mol) of L(+) mandelic acid were stirred in a reaction flask fitted with a stirrer, a thermometer and a reflux cooler for about 2.5 hours, at a temperature of 85°C. After cooling to 30°C, 27.6 grams (0.25 mol) of HCl, 33% aqueous solution, was dosed to the reaction mixture in 1 hour. The hydrochloric salt of D(-)phenylglycineamide formed was filtered and washed on the filter with 2 x 25 ml of methylisobutylketone. After drying, 44.5 grams of filtered product was obtained.
Yield = 95.4%
enantiomeric excess = 97.2 %
phenylglycineamide content (potentiometrically determined) = 80.1% (theoretical value 80.4%)

### Example II

180 grams (1.8 mol) of methylisobutylketone, 18.12 grams (0.10 mol) of DL-p-hydroxyphenylglycine methyl ester and 15.2 grams (0.10 mol) of L(+)mandelic acid were stirred in a reaction flask fitted with a stirrer, a thermometer and a reflux cooler for 4-6 hours, at a temperature of 80°C. After cooling to 30°C, 9.49 ml (0.10 mol) of HCl, 33% aqueous solution, was dosed to the reaction mixture in 1 hour. The hydrochloric salt of D(-)p-hydroxyphenylglycine-methyl ester formed was filtered and washed on the filter with 2*25 ml of methylisobutylketone. After drying, 16.3 grams of filtered D(-)-p-hydroxyphenylglycine-methyl ester.HCl salt was obtained.
Yield = 89.9%
enantiomeric excess = 92.4%

## Claims

1. Process for the preparation of an hydrochloric acid salt of an optically active phenylglycine derivative in which in an asymmetric transformation a diastereoisomeric salt is formed of the optically active phenylglycine derivative and an optically active acid and in which the resulting mixture is subsequently treated with an equivalent amount of hydrochloric acid relative to the amount of diastereoisomeric salt without intermediate isolation of the diastereoisomeric salt and in which the mother liquor containing the optically active acid and remaining after recovery of the hydrochloric acid salt of the phenylglycine derivative, is reused in the asymmetric transformation process.

2. Process according to claim 1, in which a phenylglycineamide or an ester of a phenylglycine is used as the phenylglycine derivative.

3. Process according to claim 1 or claim 2, in which phenylglycineamide or p-hydroxyphenylglycineamide is used as the phenylglycine derivative.

4. Process according to any one of claims 1-3, in which the optically active acid is optically active mandelic acid, tartaric acid, 2-pyrrolidone-5-carboxylic acid or N-acetylphenylglycine.

5. Process according to any one of claims 1-4, in which in the asymmetric transformation a mixture of the L- and D- enantiomers of the phenylglycine derivative is partly or entirely converted into the diastereoisomeric salt in the presence of a carbonyl compound with the aid of the optically active acid.

6. Process according to any one of claims 1-4, in which in the asymmetric transformation a mixture of the L- and D-enantiomers of a Schiff base of the phenylglycine derivative is partly or entirely converted into the diastereoisomeric salt with the aid of the optically active acid.

## Patentansprüche

1. Verfahren für die Herstellung eines Salzsäuresalzes eines optisch aktiven Phenylglycinderivats, wobei in einer asymmetrischen Umwandlung ein diastereoisomeres Salz aus dem optisch aktiven Phenylglycinderivat und einer optisch aktiven Säure gebildet wird und wobei das sich ergebende Gemisch nachfolgend mit einer äquivalenten Menge an Salzsäure bezogen auf die Menge an diastereoisomerem Salz ohne Zwischenisolierung des diastereoisomeren Salzes behandelt wird und wobei die Ausgangslösung, welche die optisch aktive Säure enthält und nach Gewinnung des Salzsäuresalzes des Phenylglycinderivats übrigbleibt, in dem asymmetrischen Umwandlungsverfahren wiederverwendet wird.

2. Verfahren gemäß Anspruch 1, wobei ein Phenylglycinamid oder ein Ester eines Phenylglycins als das Phenylglycinderivat verwendet wird.

3. Verfahren gemäß Anspruch 1 oder Anspruch 2, wobei Phenylglycinamid oder p-Hydroxyphenylglycinamid als Phenylglycinderivat verwendet wird.

4. Verfahren gemäß einem der Ansprüche 1-3, wobei die optisch aktive Säure optisch aktive Mandelsäure, Weinsäure, 2-Pyrrolidon-5-carbonsäure oder N-Acetylphenylglycin ist.

5. Verfahren gemäß einem der Ansprüche 1-4, wobei in der asymmetrischen Umwandlung ein Gemisch aus den L- und D-Enantiomeren des Phenylglycinderivats in Gegenwart einer Carbonylverbindung mit Hilfe der optisch aktiven Säure teilweise oder vollständig in das diastereoisomere Salz umgewandelt wird.

6. Verfahren gemäß einem der Ansprüche 1-4, wobei in der asymmetrischen Umwandlung ein Gemisch der L- und D-Enantiomere einer Schiff-Base des Phenylglycinderivats mit Hilfe der optisch aktiven Säure teilweise oder vollständig in das diastereoisomere Salz umgewandelt wird.

## Revendications

1. Procédé de préparation d'un sel d'acide chlorhydrique d'un dérivé de phénylglycine optiquement actif dans lequel, dans une transformation asymétrique, se forme un sel diastéréoisomère du dérivé de phénylglycine optiquement actif et d'un acide optiquement actif et dans lequel on traite ultérieurement le mélange résultant avec une quantité équivalente d'acide chlorhydrique par rapport à la quantité du sel diastéréoisomère sans isolement intermédiaire du sel diastéréoisomère et dans lequel on réutilise dans le procédé de transformation asymétrique, la liqueur mère contenant l'acide optiquement actif et restant après la récupération du sel d'acide chlorhydrique du dérivé de phénylglycine

2. Procédé selon la revendication 1, dans lequel on utilise un phénylglycineamide ou un ester d'une phénylglycine à titre de dérivé de phénylglycine.

3. Procédé selon la revendication 1 ou 2, dans lequel on utilise le phénylglycineamide ou le p-hydroxyphénylglycineamide à titre de dérivé de phénylglycine.

4. Procédé selon l'une quelconque des revendications 1 - 3, dans lequel l'acide optiquement actif est l'acide mandélique, l'acide tartrique, l'acide 2-pyrrolidone-5-carboxylique ou la N-acétylphénylglycine optiquement actif.

5. Procédé selon l'une quelconque des revendications 1 - 4, dans lequel, dans la transformation asymétrique, un mélange des énantiomères L- et D- du dérivé de phénylglycine est partiellement ou totalement transformé en le sel diastéréoisomère en présence d'un composé carbonyle, avec l'aide de l'acide optiquement actif.

6. Procédé selon l'une quelconque des revendications 1-- 4, dans lequel, dans la transformation asymétrique, un mélange des énantiomères L- et D- d'une base de Schiff du dérivé de phénylglycine est partiellement ou entièrement transformé en le sel diastéréoisomère avec l'aide de l'acide optiquement actif.
